(19) Europäisches Patentamt
European Patent Office
Office européen des brevets

(11) Veröffentlichungsnummer: **0 253 171**
**A1**

# (12) EUROPÄISCHE PATENTANMELDUNG

(21) Anmeldenummer: **87109159.1**

(22) Anmeldetag: **25.06.87**

(51) Int. Cl.⁴: **C07D 471/14**, A61K 31/435, //(C07D471/04,221:00,221:00,221:00)

(30) Priorität: **12.07.86 DE 3623533**

(43) Veröffentlichungstag der Anmeldung: **20.01.88 Patentblatt 88/03**

(84) Benannte Vertragsstaaten: **AT BE CH DE ES FR GB GR IT LI LU NL SE**

(71) Anmelder: **Beiersdorf Aktiengesellschaft**
**Unnastrasse 48**
**D-2000 Hamburg 20(DE)**

(72) Erfinder: **Cohnen, Erich, Dr.**
**Moorende 74**
**D-2155 Jork(DE)**
Erfinder: **Hofferber, Eva, Dr.**
**Emilienstrasse 47**
**D-2000 Hamburg 20(DE)**
Erfinder: **Beuttler, Thomas, Dr.**
**Hans-Lange-Strasse 8**
**D-2000 Hamburg 55(DE)**
Erfinder: **Stenzel, Wolfgang, Dr.**
**Lerchenweg 8**
**D-2057 Reinbek(DE)**

(54) **Pyrido [1,8] naphthyridinone, Verfahren zu ihrer Herstellung und ihre Verwendung sowie diese Verbindungen enthaltende Zubereitungen.**

(57) Pyrido[1,8]naphthyridinone der allgemeinen Formel I

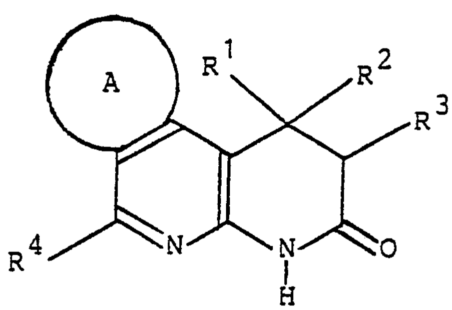

(I)

worin

$R^1$ Wasserstoff, eine geradkettige oder verzweigte Alkylgruppe mit 1 bis 6 Kohlenstoffatomen, die Phenylgruppe oder eine substituierte Phenylgruppe, eine Cycloalkylgruppe, eine Carboxygruppe, eine Alkoxycarbonylgruppe, eine Carboxamidgruppe oder Cyanogruppe,

$R^2$ Wasserstoff oder eine geradkettige oder verzweigte Alkylgruppe mit 1 bis 4 Kohlenstoffatomen

$R^3$ Wasserstoff, eine geradkettige oder verzweigte Alkylgruppe mit 1 bis 4 Kohlenstoffatomen, eine Carboxylgruppe, eine Alkoxycarbonylgruppe, eine Carboxamidgruppe oder eine Cyanogruppe

$R^4$ Wasserstoff, Halogen, die Cyanogruppe, die Hydroxylgruppe, eine Carboxamidgruppe, eine Alkylthiogruppe oder eine Alkoxygruppe mit jeweils 1 bis 6 Kohlenstoffatomen, die gegebenenfalls durch substi-

EP 0 253 171 A1

tuierte Aminogruppen substituiert sein können, eine Aryloxygruppe, eine Aminogruppe oder substituierte Aminogruppe, eine Pyrrolidino-, Piperidino-, Hydrazino-, Morpholino-, Piperazino-oder substituierte Piperazinogruppe, wobei die Alkyle jeweils geradkettig oder verzweigt sein können und

A einen Pyridinring bedeutet, wobei Ring A auch in partiell hydrierter Form vorliegen kann,

sowie ihre tautomeren Formen und ihre Salze sowie Säureadditionssalze besitzen positiv inotrope und vasodilatierende Eigenschaften und können als Arzneimittel zur Behandlung von Herzinsuffizienz, Angina pectoris und Hypertonie verwendet werden.

**Pyrido[1,8]naphthyridinone, Verfahren zu ihrer Herstellung und ihre Verwendung sowie diese Verbindungen enthaltende Zubereitungen.**

Gegenstand der Erfindung sind neue Pyrido[1,8]naphthyridinone der allgemeinen Formel I

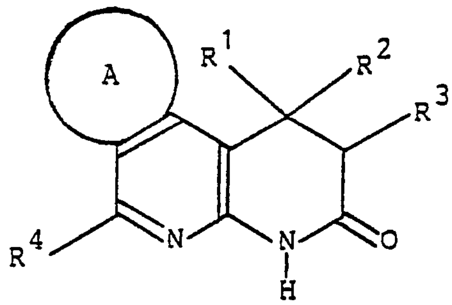

(I)

worin

$R^1$ Wasserstoff, eine geradkettige oder verzweigte Alkylgruppe mit 1 bis 6 Kohlenstoffatomen, die Phenylgruppe oder eine substituierte Phenylgruppe, eine Cycloalkylgruppe, eine Carboxylgruppe, eine Alkoxycarbonylgruppe, eine Carboxamidgruppe oder Cyanogruppe,

$R^2$ Wasserstoff oder eine geradkettige oder verzweigte Alkylgruppe mit 1 bis 4 Kohlenstoffatomen

$R^3$ Wasserstoff, eine geradkettige oder verzweigte Alkylgruppe mit 1 bis 4 Kohlenstoffatomen, eine Carboxylgruppe, eine Alkoxycarbonylgruppe, eine Carboxamidgruppe oder eine Cyanogruppe

$R^4$ Wasserstoff, Halogen, die Cyanogruppe, die Hydroxylgruppe, eine Carboxamidgruppe, eine Alkylthiogruppe oder eine Alkoxygruppe mit jeweils 1 bis 6 Kohlenstoffatomen, die gegebenenfalls durch substituierte Aminogruppen substituiert sein können, eine Aryloxygruppe, eine Aminogruppe oder substituierte Aminogruppe, eine Pyrrolidino-, Piperidino-, Hydrazino-, Morpholino-, Piperazino-oder substituierte Piperazinogruppe, wobei die Alkyle jeweils geradkettig oder verzweigt sein können und

A einen Pyridinring bedeutet, wobei Ring A auch in partiell hydrierter Form vorliegen kann,

sowie ihre tautomeren Formen und ihre Salze sowie Säureadditionssalze, Verfahren zu ihrer Herstellung und ihre Verwendung und Zubereitungen, die diese Verbindungen enthalten.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der Verbindungen der Formel I bevorzugt sind, liegen alle Salze innerhalb des Bereichs der Erfindung. Alle Salze sind wertvoll zur Herstellung der Basen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, wie beispielsweise durch Ionenaustauschverfahrensweisen, verwendet wird.

Die Verbindungen der allgemeinen Formel I und deren Salze enthalten asymmetrische Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Racemate können nach an sich bekannten Methoden in ihre optischen Antipoden aufgetrennt werden.

Bevorzugt werden Verbindungen der Formel I mit aromatischem A-Ring, in der mindestens einer der Substituenten $R^1$, $R^2$, $R^3$ oder $R^4$ einen Rest bedeutet, der nicht Wasserstoff ist, wobei, wenn $R^4$ Brom ist, auch mindestens einer der Substituenten $R^1$, $R^2$ oder $R^3$ einen Rest bedeutet, der nicht Wasserstoff ist.

Besonders bevorzugt werden Verbindungen der Formeln Ia und Ib

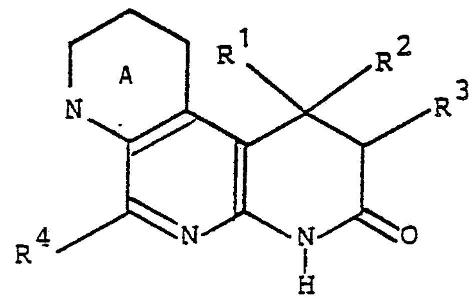

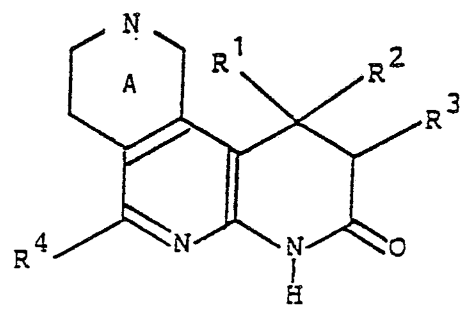

(Ia) (Ib)

in der $R^1$, $R^2$, $R^3$, $R^4$ und A die oben angegebene Bedeutung haben. Insbesondere ist in diesem Fall A Pyrido und damit aromatisch, entsprechend Formel Ia' und Ib'.

Die erfindungsgemäßen Alkylgruppen sowie die Alkylteile anderer Gruppen wie der Alkylthio-, der Ester-und Alkoxygruppen können geradkettig oder verzweigt sein und sind vorzugsweise Methyl-, Ethyl-, Propyl-und Butylgruppen. Aryl ist vorzugsweise carbocyclisches Aryl.

Halogen ist Fluor, Chlor, Brom oder Jod, vorzugsweise Brom.

Die Substituenten $R^1$ und/oder $R^2$ sind vorzugsweise Alkyl, insbesondere Methyl, Ethyl, Propyl und Isopropyl.

Bevorzugte Cycloalkylgruppen sind carbocyclische Cycloalkylgruppen wie die Cyclopropyl-, Cyclobutyl-, Cyclopentyl-und die Cyclohexylgruppe.

Alkoxycarbonylgruppen $R^1$ und $R^3$ sind vorzugsweise niedere Estergruppen, insbesondere Methoxy-, Ethoxy und Propoxycarbonylgruppen.

Die Substituenten $R^2$ und $R^3$ sind vorzugsweise Wasserstoff.

$R^2$ ist vorzugsweise dann Wasserstoff, wenn $R^1$ einen anderen Rest als Wasserstoff bedeutet oder Methyl.

Bevorzugte Reste $R^4$ sind Halogen, vorzugsweise Chlor, Brom, Cyano-, Hydroxy-, Alkoxy-, insbesondere Methoxy-und Ethoxygruppen und insbesondere die Aminogruppe und substituierte Aminogruppen.

Aryloxygruppen sind vorzugsweise carbocyclische Aryloxygruppen, insbesondere Phenoxygruppen.

Bevorzugte substituierte Aminogruppen, die Substituenten der Alkylthio-oder Alkoxygruppen bilden, sind mono-oder dialkylierte Aminogruppen mit bis zu 6, vorzugsweise bis zu 3 Kohlenstoffatomen je Alkylrest.

Substituierte Aminogruppen $R^4$ sind Acylaminogruppen oder mono-oder vorzugsweise disubstituierte Aminogruppen, insbesondere Alkylaminogruppen. Diese Alkylgruppen können geradkettig oder verzweigt sein und enthalten 1 bis 6, vorzugsweise 1 bis 3 Kohlenstoffatome, und sie können ebenfalls substituiert sein. Bevorzugte Substituenten der Alkylgruppen sind carbocyclische Arylgruppen, insbesondere die Phenylgruppe. Besonders bevorzugte Substituenten der Aminogruppe sind carbocyclische Arylalkylgruppen, insbesondere -niederalkylgruppen, vorzugsweise Benzylgruppen.

Bevorzugte Acyle der Acylaminogruppen sind Formyl und Alkylcarbonyl, insbesondere mit 1 bis 3 Kohlenstoffatomen im Alkylteil, z.B. Acetyl.

Weitere bevorzugte substituierte Aminogruppen $R^4$ sind vorzugsweise in 4-Stellung substituierte oder unsubstituierte Piperazingruppen und die Hydrazinogruppe. Vorzugsweise trägt die substituierte Piperazingruppe eine Alkoxycarbonylgruppe, insbesondere die Methoxy-, Ethoxy-oder Propoxycarbonylgruppen oder eine Benzylgruppe.

Besonders bevorzugte substituierte Aminogruppen $R^4$ sind mono-oder disubstituierte Aminogruppen $NR^5R^6$, worin $R^5$ und $R^6$, die gleich oder verschieden sein können, Wasserstoff, carbocyclische Arylalkylgruppen, verzweigte oder unverzweigte Alkylgruppen mit jeweils 1 bis 6 Kohlenstoffatomen sind, die gegebenenfalls durch Alkoxy-, Aryloxy-oder substituierte Aryloxygruppen, Pyridinylgruppen, Cycloalkylgruppen oder substituierte Aminogruppen substituiert sein können.

Vorzugsweise sind die Alkoxysubstituenten der Alkylgruppen $R^5$ und $R^6$ geradkettig oder verzweigt und besitzen insbesondere 1 bis 6, bevorzugt 1 bis 3 Kohlenstoffatome. Aryloxy ist vorzugsweise Phenoxy, bevorzugte Substituenten dafür sind geradkettige oder verzweigte Alkoxygruppen, insbesondere mit 1 bis 6, vorzugsweise 1 bis 3 Kohlenstoffatomen. Besonders bevorzugt ist die 2-Methoxyphenoxygruppe.

Substituierte Aminogruppen, die Substituenten der Alkylgruppen $R^5$ und $R^6$ bilden, sind vorzugsweise mono-oder dialkylierte Aminogruppen mit jeweils 1 bis 6, insbesondere jeweils 1 bis 3 Kohlenstoffatomen, oder cyclische Aminogruppen wie die Pyrrolidino-, Piperidino-, Morpholino-, die Piperazino-oder eine substituierte Piperazinogruppe. Bevorzugte Substituenten davon, insbesondere Piperazino-Substituenten sind Aryl-, gegebenenfalls substituierte Aryl-, insbesondere Alkoxyphenylgruppen. Vorzugsweise ist die Piperazinogruppe in 1,4-Stellung substituiert. Besonders bevorzugt ist die 2-Methoxyphenylpiperazinogruppe.

Der Ring A bildet zusammen mit den Kohlenstoffatomen der 7-und 8-Position der Bindung c des Naphthyridingerüstes einen Pyridinring, dessen Stickstoff jeweils eine der vier übrigen Positionen 7, 8, 9 und 10 des Pyridonaphthyridingerüstes besitzt und der gegebenenfalls auch partiell hydriert sein kann. Bevorzugt werden Verbindungen der Formel I, in denen das Stickstoffatom sich in der 7-Position befindet. Es sind dies die Pyrido[2,3-c][1,8]naphthyridinone der Formel Ia. Ebenfalls werden Verbindungen der Formel I bevorzugt, in denen das Stickstoffatom sich in der 9-Position befindet. Es sind dies die Pyrido[4,3-c][1,8]naphthyridinone der Formel Ib. Besonders bevorzugt werden Verbindungen der Formeln Ia oder Ib mit aromatischem Pyridorest A (Formeln Ia' und Ib').

Die 7-, 8-, 9-und 10-Position des Pyrido[1,8]naphthyridingerüstes kann vollständig, partiell oder nicht hydriert sein, so daß Doppelbindungen in 7,8-und/oder 9,10-oder 8,9-Position vorliegen können. Bevorzugt sind davon die nicht hydrierten, aromatischen Verbindungen sowie die 7-, 8-, 9-, 10-Tetrahydro-Verbindungen, insbesondere aber die aromatischen Verbindungen.

Neben den in den Beispielen genannten Verbindungen werden die folgenden erfindungsgemäßen Verbindungen und ihre Salze bevorzugt:

1-Methyl-6-brom-1,2-dihydro-pyrido[2,3-c][1,8]naphthyridin-3(4H)-on
1-Methyl-1,2-dihydro-pyrido[2,3-c][1,8]naphthyridin-3(4H)-on
1-Methyl-6-methoxy-1,2-dihydro-pyrido[2,3-c][1,8]naphthyridin-3(4H)-on
1-Methyl-6-ethoxy-1,2-dihydro-pyrido[2,3-c][1,8]naphthyridin-3(4H)-on
1-Methyl-6-propoxy-1,2-dihydro-pyrido[2,3-c][1,8]naphthyridin-3(4H)-on
1-Methyl-6-methylamino-1,2-dihydro-pyrido[2,3-c][1,8]naphthyridin-3(4H)-on
1-Methyl-6-dimethylamino-1,2-dihydro-pyrido[2,3-c][1,8]naphthyridin-3(4H)-on
1-Methyl-6-ethylamino-1,2-dihydro-pyrido[2,3-c][1,8]naphthyridin-3(4H)-on
1-Methyl-6-diethylamino-1,2-dihydro-pyrido[2,3-c][1,8]naphthyridin-3(4H)-on
1-Methyl-6-n-propylamino-1,2-dihydro-pyrido[2,3-c][1,8]naphthyridin-3(4H)-on
1-Methyl-6-isopropylamino-1,2-dihydro-pyrido[2,3-c][1,8]naphthyridin-3(4H)-on
1-Methyl-6-n-butylamino-1,2-dihydro-pyrido[2,3-c][1,8]naphthyridin-3(4H)-on
1-Methyl-6-cyano-1,2-dihydro-pyrido[2,3-c][1,8]naphthyridin-3(4H)-on
1-Methyl-6-acetamido-1,2-dihydro-pyrido[2,3-c][1,8]naphthyridin-3(4H)-on
1-Methyl-6-(4-benzyl-1-piperazino)-1,2-dihydro-pyrido[2,3-c][1,8]naphthyridin-3(4H)-on
1-Methyl-6-[2-[4-(2-methoxy-phenyl)-1-piperazinyl]ethylamino]-1,2-dihydro-pyrido[2,3-c][1,8]naphthyridin-3-(4H)-on
1-Methyl-6-carbamoyl-1,2-dihydro-pyrido[2,3-c][1,8]naphthyridin-3(4H)-on
6-Brom-1,2-dihydro-pyrido[2,3-c][1,8]naphthyridin-3(4H)-on
6-Methylamino-1,2-dihydro-pyrido[2,3-c][1,8]naphthyridin-3(4H)-on
6-Dimethylamino-1,2-dihydro-pyrido[2,3-c][1,8]naphthyridin-3(4H)-on
6-Ethylamino-1,2-dihydro-pyrido[2,3-c][1,8]naphthyridin-3(4H)-on
6-Diethylamino-1,2-dihydro-pyrido[2,3-c][1,8] naphthyridin-3(4H)-on
6-n-Propylamino-1,2-dihydro-pyrido[2,3-c][1,8]naphthyridin-3(4H)-on
6-Isopropylamino-1,2-dihydro-pyrido[2,3-c][1,8]naphthyridin-3(4H)-on
6-n-Butylamino-1,2-dihydro-pyrido[2,3-c][1,8]naphthyridin-3(4H)-on
6-Cyano-1,2-dihydro-pyrido[2,3-c][1,8]naphthyridin3(4H)-on
6-[2-[4-(2-methoxy-phenyl)-1-piperazinyl]-ethylamino]-1,2-dihydro-pyrido[2,3-c][1,8]naphthyridin-3(4H)-on
1-Isopropyl-6-brom-1,2-dihydro-pyrido[2,3-c][1,8]naphthyridin-3(4H)-on
1-Isopropyl-6-methylamino-1,2-dihydro-pyrido[2,3-c][1,8]naphthyridin-3(4H)-on
1-Isopropyl-6-dimethylamino-1,2-dihydro-pyrido[2,3-c][1,8]naphthyridin-3(4H)-on
1-Isopropyl-6-ethylamino-1,2-dihydro-pyrido[2,3-c][1,8]naphthyridin-3(4H)-on
1-Isopropyl-6-diethylamino-1,2-dihydro-pyrido[2,3-c][1,8]naphthyridin-3(4H)-on
1-Isopropyl-6-n-propylamino-1,2-dihydro-pyrido[2,3-c][1,8]naphthyridin-3(4H)-on
1-Isopropyl-6-isopropylamino-1,2-dihydro-pyrido[2,3-c][1,8]naphthyridin-3(4H)-on
1-Isopropyl-6-n-butylamino-1,2-dihydro-pyrido[2,3-c][1,8]naphthyridin-3(4H)-on
1-Isopropyl-6-cyano-1,2-dihydro-pyrido[2,3-c][1,8]naphthyridin-3(4H)-on

1-Isopropyl-6-[2-[4-(2-methoxy-phenyl)-1-piperazinyl]ethylamino]-1,2-dihydro-pyrido[2,3-c][1,8]naphthyridin-3(4H)-on
1-Methyl-6-brom-1,2-dihydro-pyrido[3,4-c][1,8]naphthyridin-3(4H)-on
1-Methyl-1,2-dihydro-pyrido[3,4-c][1,8]naphthyridin3(4H)-on
1-Methyl-6-methoxy-1,2-dihydro-pyrido[3,4-c][1,8]naphthyridin-3(4H)-on
1-Methyl-6-ethoxy-1,2-dihydro-pyrido[3,4-c][1,8]naphthyridin-3(4H)-on
1-Methyl-6-propoxy-1,2-dihydro-pyrido[3,4-c][1,8]naphthyridin-3(4H)-on
1-Methyl-6-methylamino-1,2-dihydro-pyrido[3,4-c][1,8]naphthyridin-3(4H)-on
1-Methyl-6-dimethylamino-1,2-dihydro-pyrido[3,4-c][1,8]naphthyridin-3(4H)-on
1-Methyl-6-ethylamino-1,2-dihydro-pyrido[3,4-c][1,8]naphthyridin-3(4H)-on
1-Methyl-6-diethylamino-1,2-dihydro-pyrido[3,4-c][1,8]naphthyridin-3(4H)-on
1-Methyl-6-n-propylamino-1,2-dihydro-pyrido[3,4-c][1,8]naphthyridin-3(4H)-on
1-Methyl-6-isopropylamino-1,2-dihydro-pyrido[3,4-c][1,8]naphthyridin-3(4H)-on
1-Methyl-6-n-butylamino-1,2-dihydro-pyrido[3,4-c][1,8]naphthyridin-3(4H)-on
1-Methyl-6-cyano-1,2-dihydro-pyrido[3,4-c][1,8]naphthyridin-3(4H)-on
1-Methyl-6-acetamido-1,2-dihydro-pyrido[3,4-c][1,8]naphthyridin-3(4H)-on
1-Methyl-6-(4-benzyl-1-piperazino)-1,2-dihydro-pyrido[3,4-c][1,8]naphthyridin-3(4H)-on
1-Methyl-6-[2-[4-(2-methoxy-phenyl)-1-piperazinyl]-ethylamino]-1,2-dihydro-pyrido[3,4-c][1,8]naphthyridin-3-(4H)-on
1-Methyl-6-carbamoyl-1,2-dihydro-pyrido[3,4-c][1,8]naphthyridin-3(4H)-on
6-Brom-1,2-dihydro-pyrido[3,4-c][1,8]naphthyridin-3(4H)-on
6-Methylamino-1,2-dihydro-pyrido[3,4-c][1,8]naphthyridin-3(4H)-on
6-Dimethylamino-1,2-dihydro-pyrido[3,4-c][1,8]naphthyridin-3(4H)-on
6-Ethylamino-1,2-dihydro-pyrido[3,4-c][1,8]naphthyridin-3(4H)-on
6-Diethylamino-1,2-dihydro-pyrido[3,4-c][1,8]naphthyridin-3(4H)-on
6-n-Propylamino-1,2-dihydro-pyrido[3,4-c][1,8]naphthyridin-3(4H)-on
6-Isopropylamino-1,2-dihydro-pyrido[3,4-c][1,8]naphthyridin-3(4H)-on
6-n-Butylamino-1,2-dihydro-pyrido[3,4-c][1,8]naphthyridin-3(4H)-on
6-Cyano-1,2-dihydro-pyrido[3,4-c][1,8]naphthyridin-3(4H)-on
6-[2-[4-(2-methoxy-phenyl)-1-piperaziny]-ethylamino]1,2-dihydro-pyrido[3,4-c][1,8]naphthyridin-3(4H)-on
1-Isopropyl-6-brom-1,2-dihydro-pyrido[3,4-c][1,8]naphthyridin-3(4H)-on
1-Isopropyl-6-methylamino-1,2-dihydro-pyrido[3,4-c][1,8]naphthyridin-3(4H)-on
1-Isopropyl-6-dimethylamino-1,2-dihydro-pyrido[3,4-c][1,8]naphthyridin-3(4H)-on
1-Isopropyl-6-ethylamino-1,2-dihydro-pyrido[3,4-c][1,8]naphthyridin-3(4H)-on
1-Isopropyl-6-diethylamino-1,2-dihydro-pyrido[3,4-c][1,8]naphthyridin-3(4H)-on
1-Isopropyl-6-n-propylamino-1,2-dihydro-pyrido[3,4-c][1,8]naphthyridin-3(4H)-on
1-Isopropyl-6-isopropylamino-1,2-dihydro-pyrido[3,4-c][1,8]naphthyridin-3(4H)-on
1-Isopropyl-6-n-butylamino-1,2-dihydro-pyrido[3,4-c][1,8]naphthyridin-3(4H)-on
1-Isopropyl-6-cyano-1,2-dihydro-pyrido[3,4-c][1,8]naphthyridin-3(4H)-on
1-Isopropyl-6-[2-[4-(2-methoxy-phenyl)-1-piperazinyl]ethylamino]-1,2-dihydro-pyrido[3,4-c][1,8]naphthyridin-3(4H)-on
1-Methyl-6-brom-1,2-dihydro-pyrido[4,3-c][1,8]naphthyridin-3(4H)-on
1-Methyl-1,2-dihydro-pyrido[4,3-c][1,8]naphthyridin-3(4H)-on
1-Methyl-6-methoxy-1,2-dihydro-pyrido[4,3-c][1,8]naphthyridin-3(4H)-on
1-Methyl-6-ethoxy-1,2-dihydro-pyrido[4,3-c][1,8]naphthyridin-3(4H)-on
1-Methyl-6-propoxy-1,2-dihydro-pyrido[4,3-c][1,8]naphthyridin-3(4H)-on
1-Methyl-6-methylamino-1,2-dihydro-pyrido[4,3-c][1,8]naphthyridin-3(4H)-on
1-Methyl-6-dimethylamino-1,2-dihydro-pyrido[4,3-c][1,8]naphthyridin-3(4H)-on
1-Methyl-6-ethylamino-1,2-dihydro-pyrido[4,3-c][1,8]naphthyridin-3(4H)-on
1-Methyl-6-diethylamino-1,2-dihydro-pyrido[4,3-c][1,8]naphthyridin-3(4H)-on
1-Methyl-6-n-propylamino-1,2-dihydro-pyrido[4,3-c][1,8]naphthyridin-3(4H)-on
1-Methyl-6-isopropylamino-1,2-dihydro-pyrido[4,3-c][1,8]naphthyridin-3(4H)-on
1-Methyl-6-n-butylamino-1,2-dihydro-pyrido[4,3-c][1,8]naphthyridin-3(4H)-on
1-Methyl-6-cyano-1,2-dihydro-pyrido[4,3-c][1,8]naphthyridin-3(4H)-on
1-Methyl-6-acetamido-1,2-dihydro-pyrido[4,3-c][1,8]naphthyridin-3(4H)-on
1-Methyl-6-(4-benzyl-1-piperazino)-1,2-dihydro-pyrido[4,3-c][1,8]naphthyridin-3(4H)-on
1-Methyl-6-[2-[4-(2-methoxy-phenyl)-1-piperazinyl]-ethylamino]-1,2-dihydro-pyrido[4,3-c][1,8]naphthyridin-3-(4H)-on

1-Methyl-6-carbamoyl-1,2-dihydro-pyrido[4,3-c][1,8]naphthyridin-3(4H)-on
6-Brom-1,2-dihydro-pyrido[4,3-c][1,8]naphthyridin-3(4H)-on
6-Methylamino-1,2-dihydro-pyrido[4,3-c][1,8]naphthyridin-3(4H)-on
6-Dimethylamino-1,2-dihydro-pyrido[4,3-c][1,8]naphthyridin-3(4H)-on
6-Ethylamino-1,2-dihydro-pyrido[4,3-c][1,8]naphthyridin-3(4H)-on
6-Diethylamino-1,2-dihydro-pyrido[4,3-c][1,8]naphthyridin-3(4H)-on
6-n-Propylamino-1,2-dihydro-pyrido[4,3-c][1,8]naphthyridin-3(4H)-on
6-Isopropylamino-1,2-dihydro-pyrido[4,3-c][1,8]naphthyridin-3(4H)-on
6-n-Butylamino-1,2-dihydro-pyrido[4,3-c][1,8]naphthyridin-3(4H)-on
6-Cyano-1,2-dihydro-pyrido[4,3-c]][1,8]naphthyridin-3(4H)-on
6-[2-[4-(2-methoxy-phenyl)-1-piperaziny]-ethylamino]-1,2-dihydro-pyrido[4,3-c][1,8]naphthyridin-3(4H)-on
1-Isopropyl-6-brom-1,2-dihydro-pyrido[4,3-c][1,8]naphthyridin-3(4H)-on
1-Isopropyl-6-methylamino-1,2-dihydro-pyrido[4,3-c][1,8]naphthyridin-3(4H)-on
1-Isopropyl-6-dimethylamino-1,2-dihydro-pyrido[4,3-c][1,8]naphthyridin-3(4H)-on
1-Isopropyl-6-ethylamino-1,2-dihydro-pyrido[4,3-c][1,8]naphthyridin-3(4H)-on
1-Isopropyl-6-diethylamino-1,2-dihydro-pyrido[4,3-c][1,8]naphthyridin-3(4H)-on
1-Isopropyl-6-n-propylamino-1,2-dihydro-pyrido[4,3-c][1,8]naphthyridin-3(4H)-on
1-Isopropyl-6-isopropylamino-1,2-dihydro-pyrido[4,3-c][1,8]naphthyridin-3(4H)-on
1-Isopropyl-6-n-butylamino-1,2-dihydro-pyrido[4,3-c][1,8]naphthyridin-3(4H)-on
1-Isopropyl-6-cyano-1,2-dihydro-pyrido[4,3-c][1,8]naphthyridin-3(4H)-on
1-Isopropyl-6-[2-[4-(2-methoxy-phenyl)-1-piperazinyl]-ethylamino]-1,2-dihydro-pyrido[4,3-c][1,8]naphthyridin-3(4H)-on
1-Methyl-6-brom-1,2-dihydro-pyrido[3,2-c][1,8]naphthyridin-3(4H)-on
1-Methyl-1,2-dihydro-pyrido[3,2-c][1,8]naphthyridin-3(4H)-on
1-Methyl-6-methoxy-1,2-dihydro-pyrido[3,2-c][1,8]naphthyridin-3(4H)-on
1-Methyl-6-ethoxy-1,2-dihydro-pyrido[3,2-c][1,8]naphthyridin-3(4H)-on
1-Methyl-6-propoxy-1,2-dihydro-pyrido[3,2-c][1,8]naphthyridin-3(4H)-on
1-Methyl-6-methylamino-1,2-dihydro-pyrido[3,2-c][1,8]naphthyridin-3(4H)-on
1-Methyl-6-dimethylamino-1,2-dihydro-pyrido[3,2-c][1,8]naphthyridin-3(4H)-on
1-Methyl-6-ethylamino-1,2-dihydro-pyrido[3,2-c][1,8]naphthyridin-3(4H)-on
1-Methyl-6-diethylamino-1,2-dihydro-pyrido[3,2-c][1,8]naphthyridin-3(4H)-on
1-Methyl-6-n-propylamino-1,2-dihydro-pyrido[3,2-c][1,8]naphthyridin-3(4H)-on
1-Methyl-6-isopropylamino-1,2-dihydro-pyrido[3,2-c][1,8]naphthyridin-3(4H)-on
1-Methyl-6-n-butylamino-1,2-dihydro-pyrido[3,2-c][1,8]naphthyridin-3(4H)-on
1-Methyl-6-cyano-1,2-dihydro-pyrido[3,2-c][1,8]naphthyridin-3(4H)-on
1-Methyl-6-acetamido-1,2-dihydro-pyrido[3,2-c][1,8]naphthyridin-3(4H)-on
1-Methyl-6-(4-benzyl-1-piperazino)-1,2-dihydro-pyrido[3,2-c][1,8]naphthyridin-3(4H)-on
1-Methyl-6-[2-[4-(2-methoxy-phenyl)-1-piperazinyl]ethylamino]-1,2-dihydro-pyrido[3,2-c][1,8]naphthyridin-3-(4H)-on
1-Methyl-6-carbamoyl-1,2-dihydro-pyrido[3,2-c][1,8]naphthyridin-3(4H)-on
6-Brom-1,2-dihydro-pyrido[3,2-c][1,8]naphthyridin-3(4H)-on
6-Methylamino-1,2-dihydro-pyrido[3,2-c][1,8]naphthyridin-3(4H)-on
6-Dimethylamino-1,2-dihydro-pyrido[3,2-c][1,8]naphthyridin-3(4H)-on
6-Ethylamino-1,2-dihydro-pyrido[3,2-c][1,8]naphthyridin-3(4H)-on
6-Diethylamino-1,2-dihydro-pyrido[3,2-c][1,8]naphthyridin-3(4H)-on
6-n-Propylamino-1,2-dihydro-pyrido[3,2-c][1,8]naphthyridin-3(4H)-on
6-Isopropylamino-1,2-dihydro-pyrido[3,2-c][1,8]naphthyridin-3(4H)-on
6-n-Butylamino-1,2-dihydro-pyrido[3,2-c][1,8]naphthyridin-3(4H)-on
6-Cyano-1,2-dihydro-pyrido[3,2-c][1,8]naphthyridin-3(4H)-on
6-[2-[4-(2-methoxy-phenyl)-1-piperazinyl]-ethylamino]-1,2-dihydro-pyrido[3,2-c][1,8]naphthyridin-3(4H)-on
1-Isopropyl-6-brom-1,2-dihydro-pyrido-[3,2-c][1,8]naphthyridin-3(4H)-on
1-Isopropyl-6-methylamino-1,2-dihydro-pyrido[3,2-c][1,8]naphthyridin-3(4H)-on
1-Isopropyl-6-dimethylamino-1,2-dihydro-pyrido[3,2-c][1,8]naphthyridin-3(4H)-on
1-Isopropyl-6-ethylamino-1,2-dihydro-pyrido[3,2-c][1,8]naphthyridin-3(4H)-on
1-Isopropyl-6-diethylamino-1,2-dihydro-pyrido[3,2-c][1,8]naphthyridin-3(4H)-on
1-Isopropyl-6-n-propylamino-1,2-dihydro-pyrido[3,2-c][1,8]naphthyridin-3(4H)-on
1-Isopropyl-6-isopropylamino-1,2-dihydro-pyrido[3,2-c][1,8]naphthyridin-3(4H)-on
1-Isopropyl-6-n-butylamino-1,2-dihydro-pyrido[3,2-c][1,8]naphthyridin-3(4H)-on

1-Isopropyl-6-cyano-1,2-dihydro-pyrido[3,2-c][1,8]naphthyridin-3(4H)-on
1-Isopropyl-6-[2-[4-(2-methoxy-phenyl)-1-piperazinyl]-ethylamino]-1,2-dihydro-pyrido[3,2-c][1,8]naphthyridin-3(4H)-on
1-Methyl-6-phenoxy-1,2-dihydro-pyrido[4,3-c][1,8]naphthyridin-3(4H)-on
1-n-Hexyl-6-brom-1,2-dihydro-pyrido[2,3-c][1,8]naphthyridin-3(4H)-on
1-Cyclohexyl-6-isopropylamino-1,2-dihydro-pyrido[4,3-c][1,8]naphthyridin-3(4H)-on
1,1-Dimethyl-6-methylamino-1,2-dihydro-pyrido[4,3-c][1,8]naphthyridin-3(4H)-on
1-Methyl-2-carbamoyl-6-brom-1,2-dihydro-pyrido[4,3-c][1,8]naphthyridin-3(4H)-on
1-Methyl-2-ethyl-6-methylamino-1,2-dihydro-pyrido[4,3-c][1,8]naphthyridin-3(4H)on
1-Methyl-6-hydroxy-1,2-dihydro-pyrido[2,3-c][1,8]naphthyridin-3(4H)-on
1-Phenyl-6-brom-1,2-dihydro-pyrido[2,3-c][1,8]naphthyridin-3(4H)-on
1-Ethoxycarbonyl-6-brom-1,2-dihydro-pyrido[2,3-c][1,8]naphthyridin-3(4H)-on
1-Carboxy-6-brom-1,2-dihydro-pyrido[2,3-c][1,8]naphthyridin-3(4H)-on
1-Carbamoyl-6-brom-1,2-dihydro-pyrido[2,3-c][1,8]naphthyridin-3(4H)-on
1-Cyano-6-brom-1,2-dihydro-pyrido[2,3-c][1,8]naphthyridin-3(4H)-on
1-Methyl-2-carboxy-6-brom-1,2-dihydro-pyrido[4,3-c][1,8]naphthyridin-3(4H)-on
1-Methyl-2-methoxycarbonyl-6-brom-1,2-dihydro-pyrido[4,3-c][1,8]naphthyridin-3(4H)-on
1-Methyl-2-cyano-6-brom-1,2-dihydro-pyrido[4,3-c][1,8]naphthyridin-3(4H)-on
1-Methyl-6-methylsulfido-1,2-dihydro-pyrido[2,3-c][1,8]naphthyridin-3(4H)-on
1-Methyl-6-(2-dimethylamino-ethoxy)-1,2-dihydro-pyrido[2,3-c][1,8]naphthyridin-3(4H)-on
1-Methyl-6-(2-dimethylamino-ethylsulfido)-1,2-dihydropyrido[2,3-c][1,8]naphthyridin-3(4H)-on
1-Methyl-6-amino-1,2-dihydro-pyrido[2,3-c][1,8]naphthyridin-3(4H)-on
1-Methyl-6-pyrrolidino-1,2-dihydro-pyrido[2,3-c][1,8]naphthyridin-3(4H)-on
1-Methyl-6-piperidino-1,2-dihydro-pyrido[2,3-c][1,8]naphthyridin-3(4H)-on
1-Methyl-6-hydrazino-1,2-dihydro-pyrido[2,3-c][1,8]naphthyridin-3(4H)-on
1-Methyl-6-morpholino-1,2-dihydro-pyrido[2,3-c][1,8]naphthyridin-3(4H)-on
1-Methyl-6-piperazino-1,2-dihydro-pyrido[2,3-c][1,8]naphthyridin-3(4H)-on

Die folgenden Verbindungen der allgemeinen Formel I und deren Salze mit hohem therapeutischen Effekt werden besonders bevorzugt, und zwar in der Form der Racemate sowie in der Form optisch aktiver Isomerer:

1-Methyl-6-brom-1,2-dihydro-pyrido[2,3-c][1,8]naphthyridin-3(4H)-on
1-Methyl-6-methylamino-1,2-dihydro-pyrido[2,3-c][1,8]naphthyridin-3(4H)-on
1-Methyl-6-ethylamino-1,2-dihydro-pyrido[2,3-c][1,8]naphthyridin-3(4H)-on
1-Methyl-6-n-propylamino-1,2-dihydro-pyrido[2,3-c][1,8]naphthyridin-3(4H)-on
1-Methyl-6-isopropylamino-1,2-dihydro-pyrido[2,3-c][1,8]naphthyridin-3(4H)-on
1-Methyl-6-dimethylamino-1,2-dihydro-pyrido[2,3-c][1,8]naphthyridin-3(4H)-on
1-Methyl-6-diethylamino-1,2-dihydro-pyrido[2,3-c][1,8]naphthyridin-3(4H)-on
1-Methyl-6-di-n-propylamino-1,2-dihydro-pyrido[2,3-c][1,8]naphthyridin-3(4H)-on
1-Methyl-6-diisopropylamino-1,2-dihydro-pyrido[2,3-c][1,8]naphthyridin-3(4H)-on
1-Methyl-6-[2-[4-(2-methoxy-phenyl)-1-piperazinyl]-ethylamino]-1,2-dihydro-pyrido[2,3-c][1,8]naphthyridin-3-(4H)-on
1-Methyl-6-cyano-1,2-dihydro-pyrido[2,3-c][1,8]naphthyridin-3(4H)-on
1-Methyl-6-brom-1,2-dihydro-pyrido[4,3-c][1,8]naphthyridin-3(4H)-on
1-Methyl-6-methylamino-1,2-dihydro-pyrido[4,3-c][1,8]naphthyridin-3(4H)-on
1-Methyl-6-ethylamino-1,2-dihydro-pyrido[4,3-c][1,8]naphthyridin-3(4H)-on
1-Methyl-6-n-propylamino-1,2-dihydro-pyrido[4,3-c][1,8]naphthyridin-3(4H)-on
1-Methyl-6-isopropylamino-1,2-dihydro-pyrido[4,3-c][1,8]naphthyridin-3(4H)-on
1-Methyl-6-dimethylamino-1,2-dihydro-pyrido[4,3-c][1,8]naphthyridin-3(4H)-on
1-Methyl-6-diethylamino-1,2-dihydro-pyrido[4,3-c][1,8]naphthyridin-3(4H)-on
1-Methyl-6-di-n-propylamino-1,2-dihydro-pyrido[4,3-c][1,8]naphthyridin-3(4H)-on
1-Methyl-6-diisopropylamino-1,2-dihydro-pyrido[4,3-c][1,8]naphthyridin-3(4H)-on
1-Methyl-6-cyano-1,2-dihydro-pyrido[4,3-c][1,8]naphthyridin-3(4H)-on
1-Methyl-6-[2-[4-(2-methoxy-phenyl)-1-piperazinyl]-ethylamino]-1,2-dihydro-pyrido[4,3-c][1,8]naphthyridin-3-(4H)-on

Die erfindungsgemäßen Verbindungen der Formel I sowie deren Isomere, ihre physiologisch verträglichen Salze und Säureadditionssalze sind therapeutische Wirkstoffe, besitzen hohe pharmakologische Wirkung und sind wertvolle Arzneimittel. Insbesondere zeigen sie cardiovasculäre Wirkungen. Sie besitzen positiv inotrope und vasodilatierende Eigenschaften und eignen sich deshalb zur Behandlung der Herzinsuffizienz, insbesondere der chronischen Herzinsuffizienz, der Angina pectoris und/oder der Hypertonie.

Weiterhin können die Verbindungen zur Herabsetzung der Metastasenbildung und zur Metastasenhemmung verwendet werden. Außerdem können sie zur Behandlung und Prophylaxe von Migräne, Psoriasis und Asthma, insbesondere Asthma bronchiale, dienen. Die Verbindungen hemmen die Thrombozytenaggregation. Sie können zur Behandlung und Prophylaxe von thromboembolischen Erkrankungen dienen.

Nach i.v.-Applikation in Dosierungen von 0.03 mg/kg - 3 mg/kg am Schwein und i.v.-Applikation von 0.1 mg/kg - 1.0 mg/kg sowie i. d. Applikation von 0.1 mg/kg - 10 mg/kg an der Katze kommt es zu einer länger anhaltenden blutdrucksenkenden Wirkung, die von einem anhaltenden positiv inotropen Effekt begleitet ist, der sich auch in vitro am Papillarmuskel oder Atrium des Meerschweinchens nachweisen läßt.

Die Verbindungen der vorliegenden Erfindung können oral oder parenteral angewendet werden. Die Einzeldosierungen für die orale Anwendung liegen beim Menschen bei 0,1 - 20 mg, vorzugsweise 0,5 - 10 mg, insbesondere 1 - 5 mg. Diese Dosierungen sind vorteilhaft zur Behandlung der vorstehend genannten Krankheiten, insbesondere der Herzinsuffizienz.

Die Behandlung der Psoriasis kann auch topisch erfolgen. Zur Behandlung sind beispielsweise Cremes, Lotionen, Salben, Lösungen oder Puder geeignet, die den Wirkstoff vorzugsweise in einer Menge von 1 - 10% enthalten. Zur Prophylaxe oder Behandlung wird die topische Zubereitung auf die Haut oder die erkrankte Haut einmal oder mehrmals täglich in dünner Schicht aufgetragen.

Gemäß der Erfindung werden pharmazeutische Zusammensetzungen geschaffen, die eine Verbindung der Formel I oder deren pharmazeutisch verträgliche Salze zusammen mit einem pharmazeutisch verträglichen Verdünnungsmittel oder Träger enthalten, sowie topische Zubereitungen damit, insbesondere Creme, Salbe, Lösung, Lotion und Puder, die in an sich bekannter Weise erhältlich sind.

Die Verbindungen gemäß der Erfindung können mit üblichen pharmazeutisch verträglichen Hilfsstoffen oder Trägern oder auch anderen Substanzen vermischt und beispielsweise zu oralen, sublingualen, parenteralen, topischen, rektalen Arzneiformen und zu Tropflösungen zum Aufbringen auf Schleimhäute verarbeitet werden. Sie können oral in Form von Tabletten, Dragees, Sirupen, Suspensionen, Hart-und Weichgelatinekapseln und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerte Verdünnungsmittel wie Calciumcarbonat, Natriumcarbonat, Lactose und Talk, Granulierungsmittel und Mittel, die den Zerfall der Tabletten bei oraler Verabreichung fördern wie Stärke oder Alginsäure, Bindemittel wie Stärke oder Gelatine, Gleitmittel wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darm-Trakt zu verzögern oder auch den Zutritt von Magensaft zu verhindern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermittel wie Methylcellulose, Tragacanth oder Natriumalginat, Netzmittel wie Lecithin, Polyoxyethylenstearat und Polyoxyethylensorbitanmonooleat und Konservierungsmittel wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 5 - 10 mg.

Die Verbindungen der Formel I werden dadurch hergestellt, daß man eine Verbindung der allgemeinen Formel II

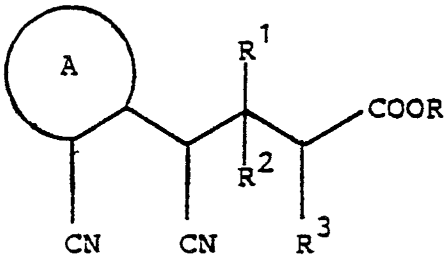

(II)

worin $R^1$, $R^2$ und $R^3$ die oben angegebene Bedeutung haben, A der Pyridinring ist und R eine niedere Alkylgruppe - bevorzugt Methyl oder Ethyl - bedeutet mit einer Halogenwasserstoffsäure, insbesondere Bromwasserstoffsäure in an sich bekannter Weise zu Verbindungen der Formel I cyclisiert, wobei $R^4$ ein Halogenatom bedeutet (F. Johnson u. W. A. Nasutaviens, J. org. Chem. **27,** 3953 (1962)). Die Cyclisierung erfolgt vorzugsweise mit HBr/Eisessig-Gemischen.

Die bevorzugten Verbindungen der Formeln Ia und Ib werden insbesondere mit den Verbindungen der Formeln III und IV erhalten, worin R, $R^1$, $R^2$ und $R^3$ die oben angegebene Bedeutung haben:

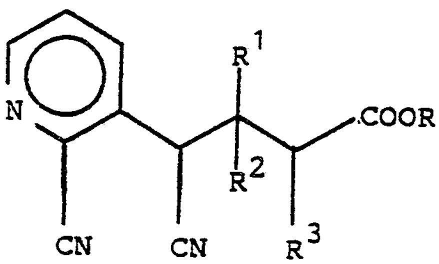

(III)

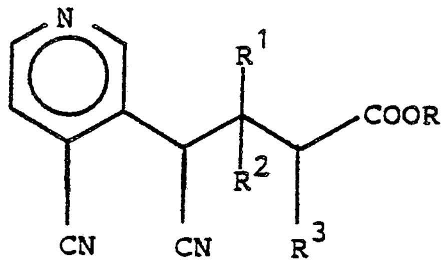

(IV)

Die Verbindungen der allgemeinen Formel I, in der $R^4$ die übrigen angegebenen Bedeutungen besitzt, sind aus den Verbindungen der Formel I mit $R^4$ = Halogen, insbesondere Brom, zugänglich.

Durch die Hydrierung mit Wasserstoff in Gegenwart eines Katalysators, insbesondere eines Palladium-Aktivkohle-Katalysators, gegebenenfalls unter Zusatz von Natriumacetat, werden die Verbindungen der Formel I erhalten, in der $R^4$ Wasserstoff bedeutet. Mit Halogeniden oder Cyaniden, insbesondere mit Alkalihalogeniden oder Alkalicyaniden, insbesondere Kaliumcyanid, lassen sich andere Halogenreste oder die Cyanogruppe in an sich bekannter Weise einführen. Mit Basen, insbesondere Alkalihydroxiden, läßt sich die Hydroxygruppe einführen. Vorzugsweise werden die vorstehend genannten Austauschreaktionen in einem Lösungsmittel bei erhöhter Temperatur durchgeführt. Durch partielle Verseifung erhält man aus der Cyanogruppe die Carboxamidgruppe.

Zur Einführung der gegebenenfalls auch substituierten Alkoxygruppen und der Aryloxygruppen erhitzt man die $R^4$ Halogen-Verbindungen mit Natrium-oder Kaliumalkoholat in dem entsprechenden Alkohol. Analog werden die gegebenenfalls auch substituierten Alkylthioverbindungen aus den entsprechenden Mercaptanen($R^4$-H) erhalten.

In der 6-Stellung substituierte Aminogruppen tragende oder 6-Amino-substituierte Verbindungen der Formel I erhält man durch Substitution von $R^4$=Halogen, durch ein obiger Definition der Aminoreste entsprechendes Amin (der Formel H-$R^4$) oder Ammoniak in alkoholischer Lösung bei Temperaturen oberhalb 100°C, gegebenenfalls unter Druck. Eingeschlossen sind hier die cyclischen Amine ($R^4$=Pyrrolidino, Piperidino, Morpholino, Piperazino, substituiertes Piperazino) und Hydrazin. Zur Herstellung der mit $R^4$ acylierten Amine setzt man Verbindungen der Formel I, in der $R^4$ Amino bedeutet mit einem Acylierungsmittel, z.B. den entsprechenden Carbonsäureanhydriden um, wobei das Acylierungsmittel als Lösungsmittel dient.

Vorzugsweise werden die Verbindungen der Formel I, in denen $R^1$ oder $R^3$ eine Carboxyl-, Carboxamid- oder Cyano-Gruppe symbolisieren, nach literaturbekannten Methoden, aus den entsprechenden Alkoxycarbonylverbindungen hergestellt. Durch Umesterung sind in an sich bekannter Weise auch andere Ester erhältlich und durch Spaltung der Ester die freien Carbonsäuren. Säureamide werden aus Estern durch Umsetzung mit Ammoniak erhalten und die Cyanogruppen enthaltenden Verbindungen durch Dehydratisierung der Amide.

Die in Ring A (Positionen 7, 8, 9, 10) partiell hydrierten Verbindungen der allgemeinen Formel I werden aus den entsprechenden aromatischen Verbindungen der Formel I durch Reduktion mit Wasserstoff in Gegenwart eines Rhodium-Katalysators bei 50-100 bar synthetisiert oder in anderen bekannten spezifischen Hydrierungsreaktionen erhalten.

Die Ausgangsverbindungen der allgemeinen Formel II können durch Michael-Addition von
2-Cyano-3-pyridyl-acetonitril bzw.
4-Cyano-3-pyridyl-acetonitril bzw.
3-Cyano-4-pyridyl-acetonitril bzw.
3-Cyano-2-pyridyl-acetonitril an Propensäureester der allgemeinen Formel V

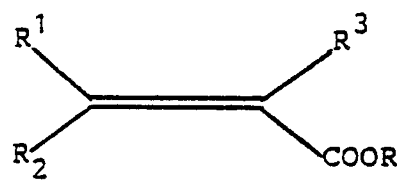

(V)

worin $R^1$, $R^2$,$R^3$ und R die angegebene Bedeutung haben, hergestellt werden. Aus 2-Cyano-3-pyridyl-acetonitril erhält man über die entsprechenden Verbindungen der Formel II (bzw. III) nach der Cyclisierung die erfindungsgemäßen Verbindungen der Formel Ia und aus 4-Cyano-3-pyridyl-acetonitril entsprechend über II (bzw. IV) die Verbindungen Ib. In gleicher Weise sind die übrigen erfindungsgemäßen Pyrido[3,4-c]-[1,8]naphthyridinone mit dem Stickstoffatom in der 8-Position und die Pyrido[3,2-c][1,8]naphthyridinone mit dem Stickstoffatom in der 10-Position synthetisierbar.

Die Propensäureester der Formel V sind bekannt oder nach bekannten Methoden erhältlich.

Die Ausgangsverbindungen der Formel II können ebenfalls durch Umsetzung der entsprechenden Cyano-acetonitril-lithiumsalze der vier vorstehend genannten Cyanopyridylacetonitrile mit 3-Brom-propionestern der allgemeinen Formel

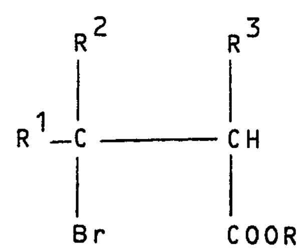

(VI)

in einem aprotischen Lösungsmittel wie z.B. Tetrahydrofuran bei Temperaturen unter 0°C dargestellt werden, wobei $R^1$, $R^2$, $R^3$ und R die angegebene Bedeutung haben.

Die Ausgangsverbindungen der Formel VI sind bekannt oder nach bekannten Verfahren erhältlich.

Die Verbindungen der allgemeinen Formel I können sowohl Basen als auch Säuren bzw. amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali-und Ammoniumsalze der Verbindungen der Formel I, die mit den entsprechenden Basen, insbesondere Natrium-, Kalium-oder Ammoniumhydroxid, erhalten werden.

Diastereoisomere können in bekannter Weise aufgrund der physikalisch-chemischen Unterschiede ihrer Bestandteile in ihre recemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bilden, Trennung der Diastereoisomeren durch fraktionierte Kristallisation und Freisetzung der Enantiomeren durch geeignete Mittel. Besonders geeignete optisch aktive Säuren beispielsweise die D-und L-Formen der Weinsäure, Ditoluoylweinsäure, Äpfelsäure, Mandelsäure, Kamphersulfonsäure oder Pyrrolidon-carbonsäure. Geeignete optisch aktive Basen sind alpha-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele erläutern die Erfindung.

Beispiel 1

3-Methyl-4-cyano-4-(2-cyano-3-pyridyl)-buttersäuremethylester

Ein Gemisch von 4,9 g (0,03 mol) 2-Cyano-3-pyridylacetonitril und 3,4 g (0,03 mol) Crotonsäuremethylester wird unter Rühren mit einer katalytischen Menge einer 5%igen methanolischen Natriummethylatlösung derart versetzt, daß die Reaktionstemperatur unter 40°C bleibt. Nach dem Abklingen der Wärmetönung wird mit verdünnter Essigsäure neutralisiert. Man extrahiert mit Chloroform, trocknet die organische Phase über $MgSO_4$, filtriert und engt in Vakuum ein. Der Rückstand wird über eine Kieselgelsäule mit Chloroform als Laufmittel gereinigt.
Ausbeute: 6,0 g (82% d. Th.)

Analog wurden die folgenden Additionsprodukte der Formel II aus 2-Cyano-3-pyridyl-acetonitril und 4-Cyano-3-pyridyl-acetonitril und den entsprechenden Propensäureestern dargestellt:
3-Isopropyl-4-cyano-4-(2-cyano-3-pyridyl)-buttersäuremethylester
3-Methyl-4-cyano-4-(4-cyano-3-pyridyl)-buttersäuremethylester
3-Isopropyl-4-cyano-4-(4-cyano-3-pyridyl)-buttersäuremethylester

Beispiel 2

4-Cyano-4-(4-cyano-3-pyridyl)-buttersäuremethylester

Eine bei minus 60°C aus 3,6g (0,036 mol) Diisopropylamin in 30 ml THF und 22,5 ml (0,036 mol) einer 1,6 n Butyllithium-Lösung in Hexan hergestellte Li-Diisopropylamid-Lösung wird bei minus 60°C in eine Lösung von 5,1 g (0,036 mol) 4-Cyano-3-pyridyl-acetonitril in 45 ml THF eingetropft.

Nach beendeter Zugabe wird dieses Reaktionsgemisch bei minus 60°C in eine Lösung von 32,6 g (0,20 mol) 3-Brompropionsäure-methylester in 45 ml THF eingebracht.

Man läßt langsam auf Raumtemperatur kommen, versetzt mit Wasser und extrahiert mit Essigsäureethylester. Nach dem Trocknen und Einengen der organischen Phase wird über eine Kieselgelsäule mit Chloroform als Laufmittel gereinigt.
Ausbeute: 6,6 g (80% d. Th.)
Analog wurde
4-Cyano-4-(2-cyano-3-pyridyl)-buttersäuremethylester dargestellt.

Beispiel 3

1-Methyl-6-brom-1,2-dihydro-pyrido[2,3-c][1,8]naphthyridin-3(4H)-on

Unter Rühren und leichter Eiskühlung werden 30,8 g (0,127 mol) 3-Methyl-4-cyano-4-(2-cyano-3-pyridyl)-buttersäuremethylester zu 145 ml 33%iger HBr-Eisessig-Lösung zugetropft. Man rührt noch 2 Stunden bei Raumtemperatur und läßt anschließend über Nacht stehen. Es fällt ein gelber Niederschlag aus. Das Reaktionsgemisch wird auf $NaHCO_3$-Lösung gegeben, der gelbe Niederschlag abgesaugt, getrocknet und anschließend aus Ethanol umkristallisiert.
Man erhält 29,7 g (80% d. Th.)
1-Methyl-6-brom-1,2-dihydro-pyrido[2,3-c][1,8]naphthyridin-3(4H)-on
Fp: 251-252°C

Analog Beispiel 3 wurden folgende erfindungsgemäße 1,2-Dihydro-pyrido[1,8]naphthyridinone der Formel VII dargestellt, in der entweder X oder Y das Pyrido-Stickstoffatom bedeutet, entsprechend Formel Ia' -(X = N, Y = CH) oder Ib' (Y = N, X = CH):

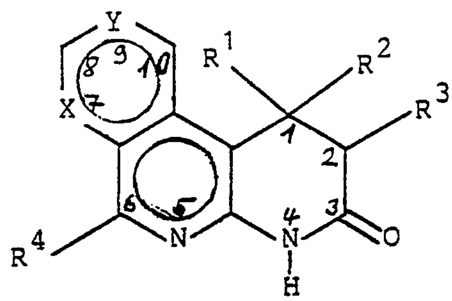

(VII)

| Beispiel Nr. | X | Y | $R^1$ | $R^2$ | $R^3$ | $R^4$ | Fp °C |
|---|---|---|---|---|---|---|---|
| 4 | N | CH | H | H | H | Br | 262 - 264 |
| 5 | N | CH | 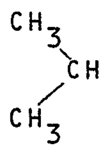 | H | H | Br | 212 - 214 |
| 6 | CH | N | H | H | H | Br | 289 |
| 7 | CH | N | $CH_3$ | H | H | Br | 265 - 267 |
| 8 | CH | N | 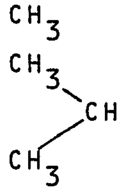 | H | H | Br | 258 - 260 |

Beispiel 9

1-Methyl-6-isopropylamino-1,2-dihydro-pyrido[4,3-c][1,8]naphthyridin-3(4H)-on

3,6 g (0,012 mol) 1-Methyl-6-brom-1,2-dihydro-pyrido[4,3-c][1,8]naphthyridin-3(4H)-on, 30 ml abs. Ethanol und 10 ml Isopropylamin werden bei 160°C 6 Stunden im Autoklaven gerührt. Nach dem Abkühlen wird der kristalline Niederschlag abgesaugt und in Methanol umkristallisiert.
Man erhält 2,82 g (87% d. Th.)
1-Methyl-6-isopropylamino-1,2-dihydro-pyrido[4,3-c][1,8]naphthyridin-3(4H)on
Fp.: 241-242°C

Beispiel 10

1-Methyl-6-[2-[4-(2-methoxy-phenyl)-1-piperazinyl]-ethylamino]-1,2-dihydro-pyrido[2,3-c][1,8]naphthyridin-3-(4H)-on

Eine Lösung aus 1,5 g (0,005 mol) 1-Methyl-6-brom-1,2-dihydro-pyrido[2,3-c][1,8]naphthyridin-3(4H)-on, 2,4 g (0,01 mol) 2-[4-(2-Methoxyphenyl)-1-piperazinyl] ethylamin, 0,5 g (0,005 mol) Triethylamin und 30 ml n-Butanol wird bei 140°C 6 Stunden lang am Rückfluß gekocht. Man engt das Reaktionsgemisch ein und verteilt den Rückstand zwischen Wasser und Chloroform. Die organische Phase wird abgetrennt, getrocknet und eingeengt. Der Rückstand wird in Petrolether/Essigsäureethylester umkristallisiert.

Man erhält 0,8 g (35,8% d. Th.)
1-Methyl-6-[2-[4-(2-methoxyphenyl)-1-piperazinyl]-ethylamino]-1,2-dihydro-pyrido[2,3-c][1,8]naphthyridin-3-(4H)-on
Analog Beispiel 9 und 10 wurden folgende erfindungsgemäße
1,2-Dihydro-pyrido[1,8]-naphthyridin-3(4H)-one der Formel VII dargestellt.

| Beispiel Nr. | x | y | $R^1$ | $R^2$ | $R^3$ | $R^4$ | Fp. °C |
|---|---|---|---|---|---|---|---|
| 11 | N | CH | $CH_3$ | H | H | $CH_3$-NH- | 250-251 |
| 12 | N | CH | $CH_3$ | H | H | 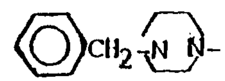 | 192-193 |
| 13 | N | CH | H | H | H | $CH_3$-NH | 287-289 |
| 14 | N | CH | H | H | H | 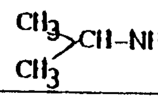 | 195-197 |
| 15 | N | CH | 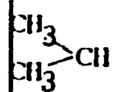 | H | H | $CH_3$NH | 235-236 |
| 16 | N | CH | 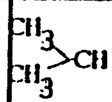 | H | H | 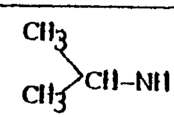 | 235-236 |
| 17 | CH | N | $CH_3$ | H | H | $CH_3$-NH | > 300 |

| | | | | | | | |
|---|---|---|---|---|---|---|---|
| 18 | CH | N | $CH_3$ | H | H | 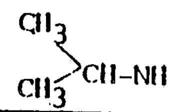 | > 300 |
| 19 | CH | N | $CH_3$ | H | H | 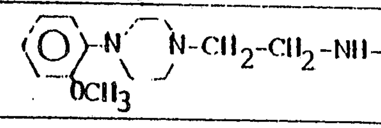 | 199-200 |
| 20 | CH | N | H | H | H | $CH_3-NH-$ | > 320 |
| 21 | CH | N | H | H | H | 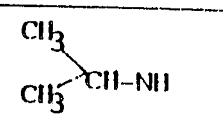 | 304-306 |
| 22 | CH | N | 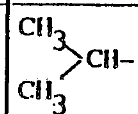 | H | H | $CH_3-NH-$ | 258-260 |
| 23 | CH | N | 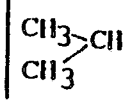 | H | H | 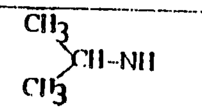 | 294-295 |

Beispiel 24

1-Methyl-6-cyano-1,2-dihydro-pyrido[2,3-c][1,8]naphthyridin-3(4H)-on

2 g (0,007 mol) 1-Methyl-6-brom-1,2-dihydro-pyrido[2,3-c][1,8]naphthyridin-3(4H)-on, 0,3 g (0,003 mol) CuCN und 0,3 g (0,005 mol) KCN werden mit 20 ml Methanol und 10 ml Wasser 7 Stunden lang bei 160°C im Autoklaven erhitzt. Nach Abkühlen wird abgesaugt und der Rückstand 4 Stunden in Chloroform/Methanol 1:1 am Rückfluß digeriert. Die organische Phase wird filtriert, getrocknet und eingeengt. Man reinigt den Rückstand durch Umkristallisation in Ethanol/Petrolether und erhält 0,5 g (60,1% d. Th.)
1-Methyl-6-cyano-1,2-dihydro-pyrido[2,3-c][1,8]naphthyridin-3(4H)-on
Fp.: über 300°C

Beispiel 25

1-Isopropyl-6-cyano-1,2-dihydro-pyrido[2,3-c][1,8]naphthyridin-3(4H)-on

1,6 g (0,005 mol) 1-Isopropyl-6-brom-1,2-dihydro-pyrido[2,3-c][1,8]naphthyridin-3(4H)-on werden mit 0,45 g (0,005 mol) CuCN bei 220°C im Schwerölbad geschmolzen. Nach 1 Stunde läßt man die Schmelze abkühlen, zerreibt den Rückstand im Mörser und verrührt mit einer konzentrierten Ammoniak-Lösung. Der Niederschlag wird abgesaugt, mit Wasser gewaschen, getrocknet und auf einer Kieselgelsäule mit Chloroform gereinigt. Man erhält 0,28 g (21% d. Th.)
1-Isopropyl-6-cyano-1,2-dihydro-pyrido[2,3-c][1,8]naphthyridin-3(4H)-on.
Fp.: 287-299°C

Beispiel 26

1-Methyl-6-carbamoyl-1,2-dihydro-pyrido[2,3-c][1,8]naphthyridin-3(4H)-on

2 g (0.008 mol) 1-Methyl-6-cyano-1,2-dihydro-pyrido[2,3-c][1,8]naphthyridin-3(4H)-on werden in 40 ml 96%iger Schwefelsäure 3 Stunden bei Raumtemperatur stehengelassen. Nach Neutralisation mit wässriger $Na_2CO_3$ -Lösung wird mehrfach mit Methylenchlorid extrahiert, die vereinigten organischen Basen werden getrocknet, dann wird im Vakuum eingedampft und der Rückstand an Kieselgel mit Chloroform/Methanol 98:2 chromatographiert.
Man erhält 1.29 g (63% d.Th.)
1-Methyl-6-carbamoyl-1,2-dihydro-pyrido[2,3-c][1,8]naphthyridin-3(4H)-on
Fp.: 229°C (Zersetzung)

Beispiel 27

1-Methyl-6-ethoxy-1,2-dihydro-pyrido[4,3-c][1,8]naphthyridin-3(4H)-on

1,5 g (0.005 mol) 1-Methyl-6-brom-1,2-dihydro-pyrido[4,3-c][1,8]naphthyridin-3(4H)-on werden mit 0,23 g (0.01 mol) Natrium in 20 ml Ethanol gelöst und bei 160°C 3 Stunden lang im Autoklaven erhitzt. Man saugt das ausgefallene Produkt ab, wäscht mit Ethanol nach und trocknet. Man erhält 0,8 g (62,2% d.Th.)
1-Methyl-6-ethoxy-1,2-dihydro-pyrido[4,3-c][1,8]naphthyridin-3(4H)-on

Beispiel 28

1-Methyl-1,2,7,8,9,10-hexahydro-pyrido[2,3-c][1,8]naphthyridin-3(4H)-on

1,5 g (,0,005 mol) 1-Methyl-6-brom-1,2-dihydro-pyrido[2,3-c][1,8]naphthyridin-3(4H)-on werden unter Zusatz von 0,4 g (0,005 mol) Na-Acetat in einem Gemisch aus 100 ml Ethanol und 100 ml Eisessig mit Pd/C (0,3 g/10%ig) bei Raumtemperatur und Atmosphärendruck 24 Stunden hydriert. Man saugt vom Katalysator ab, engt ein und kristallisiert aus Ethanol um. Man erhält 0,7 g (64,4% d. Th.)
1-Methyl-1,2,7,8,9,10-hexahydro-pyrido[2,3-c][1,8]naphthyridin-3(4H)-on
Fp.: 309-311 °C

Beispiel 29

Herstellung von Tabletten

Tabletten, die die im folgenden genannten Bestandteile enthalten, lassen sich in bekannter Weise herstellen. Sie können zur Behandlung von den vorstehend genannten Krankheiten, insbesondere der Herzinsuffizienz, in einer Dosierung von einer Tablette ein-bis zweimal täglich angewendet werden:
1-Methyl-6-isopropylamino-1,2-dihydro-pyrido[4,3-c][1,8]naphthyridin-3(4H)-on 4 mg
Lactose 75 mg
Maisstärke 10 mg
Mikrokristalline Cellulose 8 mg
Polyvinylpyrrolidon 1 mg
Magnesiumstearat 0,5 mg
hochdisperses Siliziumdioxid 0,5 mg

Beispiel 30

Mit den angegebenen Bestandteilen (Gewichtsmengen) wird eine Fettsalbe hergestellt:
Weißes Vaselin 76%
dickflüssiges Paraffin 15%
Glycerin 2%
Emulgator (Polyoxyethylen-40-stearat) 2%
1-Methyl-6-isopropylamino-1,2-dihydro-pyrido-[4,3-c][1,8]naphthyridin-3(4H)-on 5%

Vaselin, Paraffin, Glycerin und der Emulgator werden auf 80°C erhitzt. Dann wird der Wirkstoff zugegeben. Es wird bis zum Lösen gerührt und das Rühren bis zum Festwerden der Masse (salbenartige Konsistenz) fortgesetzt.

Die Salbe wird dreimal täglich angewendet.

**Ansprüche**

1. Pyrido[1,8]naphthyridinone der allgemeinen Formel I

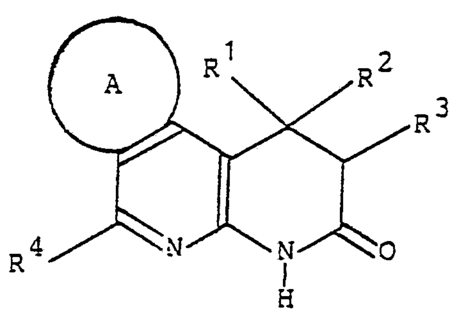

(I)

worin
$R^1$ Wasserstoff, eine geradkettige oder verzweigte Alkylgruppe mit 1 bis 6 Kohlenstoffatomen, die Phenylgruppe oder eine substituierte Phenylgruppe, eine Cycloalkylgruppe, eine Carboxylgruppe oder Cyanogruppe,

$R^2$ Wasserstoff oder eine geradkettige oder verzweigte Alkylgruppe mit 1 bis 4 Kohlenstoffatomen

$R^3$ Wasserstoff, eine geradkettige oder verzweigte Alkylgruppe mit 1 bis 4 Kohlenstoffatomen, eine Carboxylgruppe, eine Alkoxycarbonylgruppe, eine Carboxamidgruppe oder eine Cyanogruppe

$R^4$ Wasserstoff, Halogen, die Cyanogruppe, die Hydroxylgruppe, eine Carboxamidgruppe, eine Alkylthiogruppe oder eine Alkoxygruppe mit jeweils 1 bis 6 Kohlenstoffatomen, die gegebenenfalls durch substituierte Aminogruppen substituiert sein können, eine Aryloxygruppe, eine Aminogruppe oder substituierte Aminogruppe, eine Pyrrolidino-, Piperidino-, Hydrazino-, Morpholino-, Piperazino-oder substituierte Piperazinogruppe, wobei die Alkyle jeweils geradkettig oder verzweigt sein können und

A einen Pyridinring bedeutet, wobei Ring A auch in partiell hydrierter Form vorliegen kann, sowie ihre tautomeren Formen und ihre Salze sowie Säureadditionssalze.

2. Verbindungen gemäß Anspruch 1 der Formeln Ia′ und Ib′

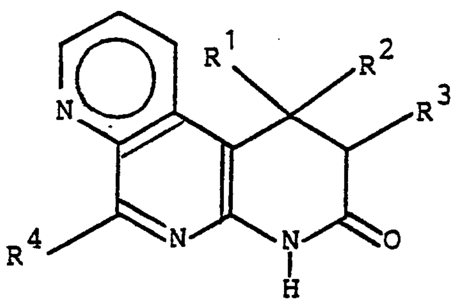

(Ia')

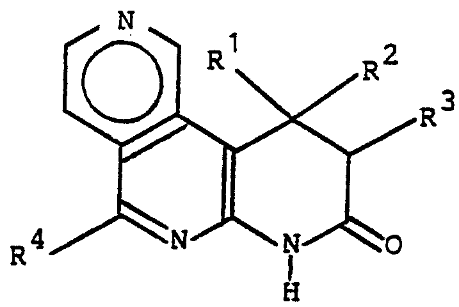

(Ib')

worin $R^1$, $R^2$, $R^3$ und $R^4$ die oben angegebene Bedeutung haben und deren Säureadditionssalze.

3. 1-Methyl-6-isopropyl-1,2-dihydro-pyrido[4,3-c][1,8]naphthyridin-3(4H)-on

4. 1-Methyl-6-brom-1,2-dihydro-pyrido[4,3-c][1,8]naphthyridin-3(4H)-on

5. 1-Methyl-6-methylamino-1,2-dihydro-pyrido[4,3-c][1,8]naphthyridin-3(4H)-on

6. 1-Methyl-6-methylamino-1,2-dihydro-pyrido[2,3-c][1,8]naphthyridin-3(4H)-on

7. Verfahren zur Herstellung der Verbindungen gemäß Anspruch 1, dadurch gekennzeichnet, daß man Verbindungen der Formel II

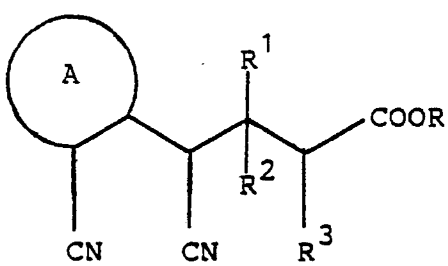

(II)

in der $R^1$, $R^2$, $R^3$ und A die oben angegebene Bedeutung haben und R eine niedere Alkylgruppe bedeutet, mit einer Halogenwasserstoffsäure in an sich bekannter Weise zu Verbindungen der Formel I cyclisiert, in der $R^4$ Halogen bedeutet und gegebenenfalls diese 6-Halogenverbindungen

a) zur Einführung des Restes $R^4$ = Wasserstoff hydriert,

b) zur Herstellung anderer 6-Halogenverbindungen mit entsprechenden Halogeniden umsetzt,

c) zur Herstellung von 6-Cyanoverbindungen mit Cyaniden,

d) zur Herstellung von 6-Hydroxyverbindungen mit Basen,

e) zur Herstellung von 6-Alkoxyverbindungen und 6-Aryloxyverbindungen mit den entsprechenden Alkoholaten,

f) zur Herstellung der 6-Aminoverbindungen mit Ammoniak,

g) zur Herstellung der Verbindungen, der Formel I, in der $R^4$ eine substituierte Aminogruppe bedeutet mit entsprechenden Aminen der Formel H-$R^4$,

h) zur Herstellung von 6-Alkylthioverbindungen mit den entsprechenden Mercaptanen umsetzt und

i) zur Herstellung der 6-Carboxamide die Cyanogruppe partiell verseift,
und gegebenenfalls Pyrido-Verbindungen der Formel I zur Herstellung der im Pyrido-Ring partiell hydrierten Verbindungen der Formel I hydriert und zur Herstellung der 6-Acylaminoverbindungen die 6-Aminoverbindungen acyliert.

8. Verfahren zur Herstellung der Verbindungen der Formel I gemäß Anspruch 1, in der $R^1$ und/oder $R^3$ die Carboxyl-, Carboxamid-oder Cyanogruppe bedeuten, dadurch gekennzeichnet, daß man die Carbonsäureestergruppen $R^1$ und/oder $R^3$ in an sich bekannter Weise zur Herstellung der Carbonsäuren spaltet, zur Herstellung der Amide mit Ammoniak umsetzt und zur Herstellung der Cyanoverbindungen die Amide dehydratisiert.

9. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

10.Verbindungen der Formel I gemäß Anspruch 1 als pharmazeutischer Wirkstoff, insbesondere zur Behandlung von Herzinsuffizienz, Angina pectoris und Hypertonie.

Europäisches Patentamt

# EUROPÄISCHER RECHERCHENBERICHT

Nummer der Anmeldung

EP 87 10 9159

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 167 045 (BEIERSDORF)<br>* Ansprüche 1,6; Seite 10, Zeilen 1-10 *<br>----- | 1,9 | C 07 D 471/14<br>A 61 K 31/435//<br>(C 07 D 471/04<br>C 07 D 221:00<br>C 07 D 221:00<br>C 07 D 221:00 ) |
| | | | **RECHERCHIERTE SACHGEBIETE (Int. Cl.4)** |
| | | | C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 15-10-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82